# EUROPEAN PATENT SPECIFICATION

(11) **EP 3 897 436 B1**
(45) Date of publication and mention of the grant of the patent: **19.06.2024**
(21) Application number: 19839028.8
(22) Date of filing: 17.12.2019
(51) Int. Cl.: A61B 18/14

(54) **STEERABLE TIP COOLED RADIOFREQUENCY ABLATION PROBE**
LENKBARE SPITZENGEKÜHLTE HOCHFREQUENZABLATIONSSONDE
SONDE D'ABLATION RADIOFRÉQUENCE REFROIDIE À UNE POINTE ORIENTABLE

(30) Priority: 21.12.2018 US 201816229065
(43) Date of publication of application: 27.10.2021
(73) Proprietor: Avent, Inc., Alpharetta, GA 30004 (US)
(72) Inventor: WANG, Ruoya, Alpharetta, Georgia 30004 (US)
(74) Representative: Potter Clarkson
(86) International application number: PCT/US2019/066690
(87) International publication number: WO 2020/131770

(56) References cited:
- WO-A1-02/28475
- US-A1- 2005 177 209
- US-A1- 2015 297 246
- US-B1- 9 956 032

## Description

### FIELD OF THE INVENTION

The present invention relates generally to a system for applying energy for the treatment of tissue, and more particularly to a cooled radiofrequency probe.

### BACKGROUND

Lower back injuries and chronic joint pain are major health problems resulting not only in debilitating conditions for the patient, but also in the consumption of a large proportion of funds allocated for health care, social assistance and disability programs. In the lower back, disc abnormalities and pain may result from trauma, repetitive use in the workplace, metabolic disorders, inherited proclivity, and/or aging. The existence of adjacent nerve structures and innervation of the disc are very important issues in respect to patient treatment for back pain. In joints, osteoarthritis is the most common form of arthritis pain and occurs when the protective cartilage on the ends of bones wears down over time.

A minimally invasive technique of delivering high-frequency electrical current has been shown to relieve localized pain in many patients. Generally, the high-frequency current used for such procedures is in the radiofrequency (RF) range, i.e. between 100 kHz and 1 GHz and more specifically between 300-600 kHz. The RF electrical current is typically delivered from a generator via connected electrodes that are placed in a patient's body, in a region of tissue that contains a neural structure suspected of transmitting pain signals to the brain. The electrodes generally include an insulated shaft with an exposed conductive tip to deliver the radiofrequency electrical current. Tissue resistance to the current causes heating of tissue adjacent resulting in the coagulation of cells (at a temperature of approximately 45°C for small unmyelinated nerve structures) and the formation of a lesion that effectively denervates the neural structure in question. Denervation refers to a procedure whereby the ability of a neural structure to transmit signals is affected in some way and usually results in the complete inability of a neural structure to transmit signals, thus removing the pain sensations. This procedure may be done in a monopolar mode where a second dispersive electrode with a large surface area is placed on the surface of a patient's body to complete the circuit, or in a bipolar mode where a second radiofrequency electrode is placed at the treatment site. In a bipolar procedure, the current is preferentially concentrated between the two electrodes.

To extend the size of a lesion, radiofrequency treatment may be applied in conjunction with a cooling mechanism, whereby a cooling means is used to reduce the temperature of the electrode-tissue interface, allowing more energy or power to be applied without causing an unwanted increase in local tissue temperature that can result in tissue desiccation, charring, or steam formation. The application of more energy or power allows regions of tissue further away from the energy delivery device to reach a temperature at which a lesion can form, thus increasing the size/volume of the lesion.

The treatment of pain using high-frequency electrical current has been applied successfully to various regions of patients' bodies suspected of contributing to chronic pain sensations. For example, with respect to back pain, which affects millions of individuals every year, high-frequency electrical treatment has been applied to several tissues, including intervertebral discs, facet joints, sacroiliac joints as well as the vertebrae themselves (in a process known as intraosseous denervation). In addition to creating lesions in neural structures, application of radiofrequency energy has also been used to treat tumors throughout the body. Further, with respect to knee pain, which also affects millions of individuals every year, high-frequency electrical treatment has been applied to several tissues, including, for example, the ligaments, muscles, tendons, and menisci.

Due to the large volume lesions generated by cooled radiofrequency probe procedures, care must be taken when treating sensitive locations, particularly around areas that cannot sustain significant collateral ablative damage. Existing cooled radiofrequency probes typically have a 17 gauge diameter, which is very large in diameter in comparison to non-cooled radiofrequency ablation probes and other nerve block needles, which may have a diameter between 18 gauge and 22 gauge, for example. As a result, the existing cooled radiofrequency probes result in puncture site and procedural pain. Additionally, the existing 17 gauge cooled radiofrequency probes have reduced steerability of the needle in tissue in comparison to non-cooled radiofrequency ablation probes and other nerve block needles having a smaller diameter. As such, existing 17 gauge cooled radiofrequency probes are more difficult to avoid obstructions, such as bones, in the patient's tissue and must be repeatedly withdrawn from the tissue and re-inserted in the direction to avoid obstructions, causing additional potential tissue damage.

Consequently, there is a need for a cooled radiofrequency ablation probe having optimized shape and size to improve the steerability of the probe needle in tissue. Moreover, a cooled radiofrequency probe that has a curved or bendable tip and/or reduced diameter in order to reduce trauma at the puncture site and pain resulting from the procedure would be useful.

US 2005/177209 discloses a medical probe assembly and system for treating tissue. The system optionally comprises an energy source, two probe assemblies, and one or more cooling devices to provide cooling to at least one of the probe assemblies. The probe assemblies may be configured in a bipolar mode, whereby current flows preferentially between the probe assemblies. The probe assemblies and system are particularly useful to deliver radio frequency energy to a patient's body. RF energy delivery may be used for various applications, including the treatment of pain, tumor ablation and cardiac ablation.

### SUMMARY OF THE INVENTION

According to a first aspect of the present invention, there is provided a cooled radiofrequency ablation probe according to claim 1. Embodiments of the ablation probe of the invention are defined in the dependent claims. The probe can be capable of creating a lesion in the patient's tissue when electrical or radiofrequency energy is applied, wherein the lesion created by the probe is of approximately the same size as a lesion created by a larger 17 gauge diameter cooled radiofrequency probe under identical temperature and power settings.

According to a second aspect of the present invention, there is provided a cooled radiofrequency ablation delivery kit according to claim 10. Embodiments of the ablation delivery kit of the invention are defined in the dependent claims.

### BRIEF DESCRIPTION OF THE DRAWINGS

A full and enabling disclosure of the present invention, including the best mode thereof, directed to one of ordinary skill in the art, is set forth in the specification, which makes reference to the appended figures, in which:
FIG. 1A illustrates a side view of an exemplary straight 20 gauge cooled radiofrequency probe;
FIG. 1B illustrates a side view of a prior art straight 17 gauge cooled radiofrequency probe;
FIGs. 2A-C illustrate a side view of a curved radiofrequency probe, a stylet, and an introducer;
FIG. 3 illustrates a side view of an assembly of the curved radiofrequency probe and introducer of FIGs. 2A-C;
FIGs. 4A-C illustrate a side view of the angles of curvature of the curved radiofrequency probe, stylet, and introducer of FIGs. 2A-C;
FIG. 5 illustrates a perspective partial cutaway view of interior components of the distal end of a cooled radiofrequency probe of the present invention;
FIG. 6 illustrates an embodiment of a cooled radiofrequency probe of the present invention; and
FIG. 7 illustrates a side view of yet another embodiment of a cooled radiofrequency probe of the present invention.

### DETAILED DESCRIPTION OF THE INVENTION

Reference will now be made in detail to one or more embodiments of the invention, examples of the invention, examples of which are illustrated in the drawings. Each example and embodiment is provided by way of explanation of the invention, and is not meant as a limitation of the invention as defined in the claims. For example, features illustrated or described as part of one embodiment may be used with another embodiment to yield still a further embodiment.

Before explaining at least one embodiment of the invention in detail, it is to be understood that the invention is not limited in its application to the details of construction and the arrangement of the components set forth in the following description or illustrated in the drawings, but is defined in the appended claims. The invention is capable of other embodiments or of being practiced or carried out in various ways within the scope as defined in the claims. Also, it is to be understood that the phraseology and terminology employed herein is for the purpose of description and should not be regarded as limiting.

For the purposes of this invention, a lesion refers to the region of tissue that has been irreversibly damaged as a result of the application of thermal energy, and the invention is not intended to be limited in this regard. Furthermore, for the purposes of this description, proximal generally indicates that portion of a device or system next to or nearer to a handle of the probe (when the device is in use), while the term distal generally indicates a portion further away from the handle of the probe (when the device is in use).

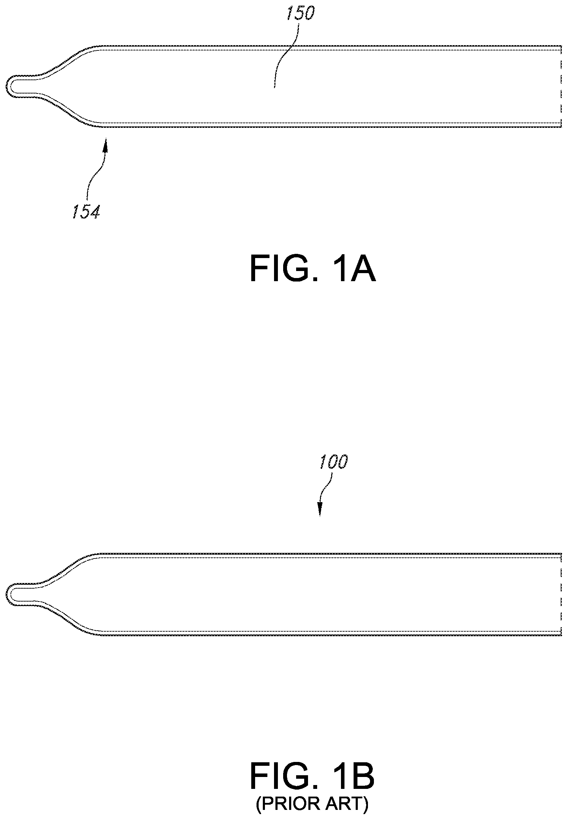

Referring now to the drawings, FIG. 1B illustrates a prior art cooled radiofrequency ablation probe 100. The probe 100 has a 17 gauge diameter and is straight, with no curvature along its length. The term "17 gauge" corresponds to a needle having an outer diameter of approximately 0.058 inches (1.473 mm). FIG. 1A illustrates, by comparison, a cooled radiofrequency probe 150 that has a 20 gauge diameter and is straight, with no curvature along its length, and terminates at a distal end 154. The term "20 gauge" corresponds to a needle having an outer diameter of approximately 0.0358 inches (0.908 mm). The diameter of the distal end 154 of the probe 150 is about 30% smaller than the diameter of the 17 gauge cooled radiofrequency probe.

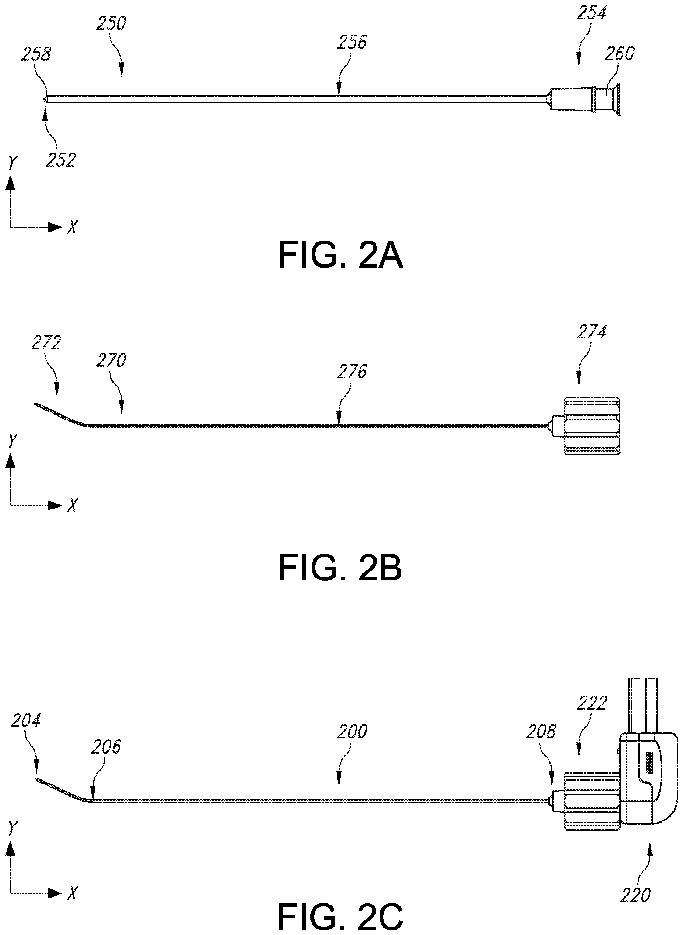

FIG. 2C illustrates the curved cooled radiofrequency probe 200, along with accessories for using the probe 200 for cooled radiofrequency ablation treatment of a patient's tissue according to an exemplary cooled radiofrequency treatment. Specifically, FIG. 2A shows a polymer introducer 250 through which the probe 200 can be inserted into a patient's tissue. The polymer introducer 250 has an elongated portion 256 extending between a distal end 252 and a proximal end 254. The polymer introducer 250 can have a distal opening 258 positioned at the distal end 252 and a connector 260 positioned at the proximal end 254. The connector 260 can be, for instance, a male luer connector. FIG. 2B also shows a 20 gauge stylet 270 having an elongated portion 276 extending between a curved distal end 272 and a connector 274. The connector 274 can be a female luer connector which can receive the connector 260 of the introducer 250 to secure the stylet 270 and introducer 250 together.

As shown in FIG. 2C, the curved probe 200 can include a handle end 208 at an opposite end from the distal end 206 where the probe 200 is configured to a cooled radiofrequency probe handle 220. The handle 220 can include a luer lock 222 for connecting to the connector 254 of the introducer 250. The luer lock 222 can be a female luer lock.

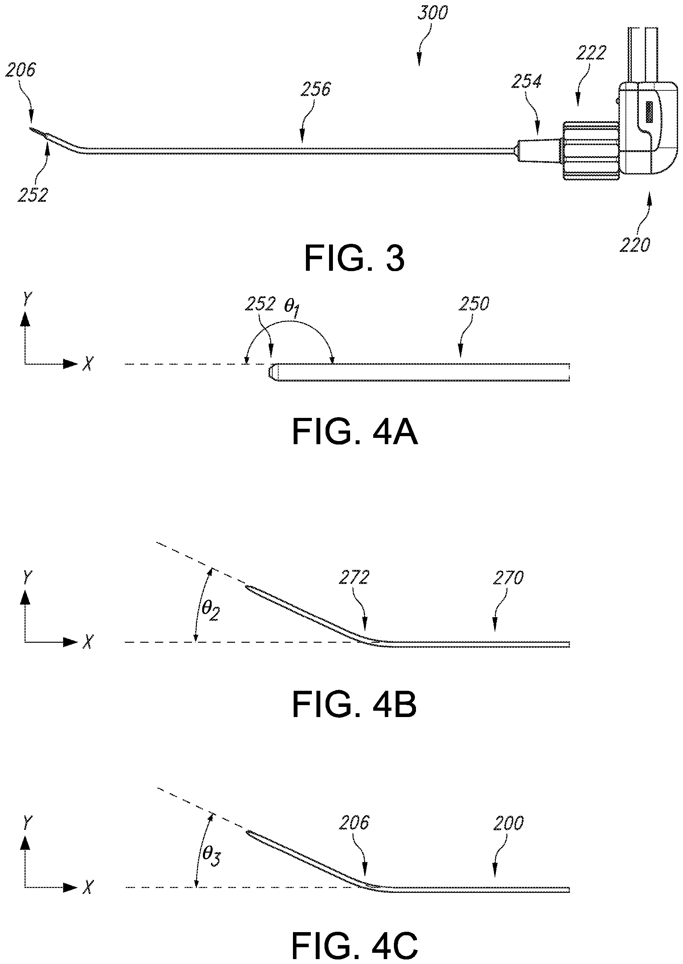

FIG. 3 illustrates an assembly 300 of the curved probe 200 inserted within the polymer introducer 250, with the male connector 260 of the polymer introducer 250 mated within the female luer lock 222 of the handle 220 of the probe 200. As shown in FIG. 3, the distal end 252 of the polymer introducer 250 can conform to the curvature of the distal end 206 of the probe 200.

Referring again to FIGs. 2A-C, the stylet 270 can be inserted through an introducer, such as the polymer introducer 250, with a distal end 272 of the stylet 270 protruding through the distal opening 258 at the distal end 252 of the introducer 250. The distal end 272 of the stylet 270 may have a needle or sharpened tip that can puncture the patient's skin and create a pathway to the target nerve location. The distal end 272 of the stylet 270 can be configured to protrude from the distal opening 258 of the introducer 250 a distance ranging from about 2 mm to about 10 mm, for example about 6 mm. After puncturing the skin, the stylet 270 can be removed from the introducer 250 with the introducer 250 left in place in the patient's tissue. Then, the cooled radiofrequency probe 200 can be inserted into the introducer 250 such that the distal end 206 of the probe 200 extends through the distal opening 258 at the distal end 252 of the introducer 250 in order to access the target nerve location. The distal end 206 of the probe 200 can be configured to protrude from the distal opening 258 of the introducer 250 a distance ranging from about 1 mm to about 7 mm, for example about 4 mm. The polymer introducer 250 can create an electrical barrier along the length of the probe 200 such that only the distal end 206 of the probe 200 that extends from the distal end 252 of the introducer 250 is electrically exposed for delivering RF energy into the tissue. In such a configuration, the portion of the probe 200 that is not exposed from the distal end 252 of the introducer 250 can be electrically insulated by the polymer introducer 250.

FIGs. 4A-C illustrate exemplary angles of curvature of the probe 200, polymer introducer 250, and stylet 270. The probe 200, polymer introducer 250 and stylet 270 can extend along a longitudinal axis x, and the angles of curvature of the probe 200, polymer introducer 250, and stylet 270 can be determined relative to the x-axis towards a transverse y-axis as shown in FIGs. 4A-C. The distal end 252 of polymer introducer 250 can be straight, and thereby can have an angle θ₁ which is equal to about 0 degrees or about 180 degrees. The polymer introducer 250 can be made from any number of polymer-based materials that possess a high dielectric and structural strength such as, but not limited to, fluorinated ethylene propylene (FEP), polytetrafluoroethylene (PTFE), high-density polyethylene (HDPE), and polyamide. Due to the flexible material of the polymer introducer 250, the straight (i.e., about 0 or 180 degree angle) polymer introducer 250 can conform to a curved tip of a probe 200 or stylet 270 inserted therein without kinking or buckling caused by the curvature. In comparison, using a conventional metal introducer (not shown) with a curved probe (e.g. probe 200) can cause kinking or buckling of the introducer and/or the probe, which can then interfere with the steerability of the probe in the patient's tissue.

The distal end 272 of the stylet 270 can extend at an angle θ₂ with respect to the longitudinal x axis, as shown in FIG. 4B. The angle θ₂ can be any angle within the range of about 0 degrees to about 30 degrees with respect to the longitudinal x axis, such as from about 1 degree to about 25 degrees, or from about 10 degrees to about 22 degrees, or from about 15 degrees to about 20 degrees. Alternatively, the angle Θz can be about 0 degrees or about 180 degrees with respect to the longitudinal x axis to form a straight stylet (not shown).

The distal end 206 of the cooled radiofrequency probe 200 can be curved at an angle θ₃ with respect to the longitudinal x axis as shown in FIG. 4C. The angle θ₃ may be in a range from about greater than 0 degrees to about 30 degrees, such as from about 1 degree to about 30 degrees, for example from about 5 degrees to about 25 degrees, such as from about 10 degrees to about 22 degrees, or from about 15 degrees to about 20 degrees. In one arrangement, the angle θ₃ may be approximately equal to the angle θ₂ of a curved stylet, e.g. the curved stylet 270, as illustrated in FIGs. 4B-C.

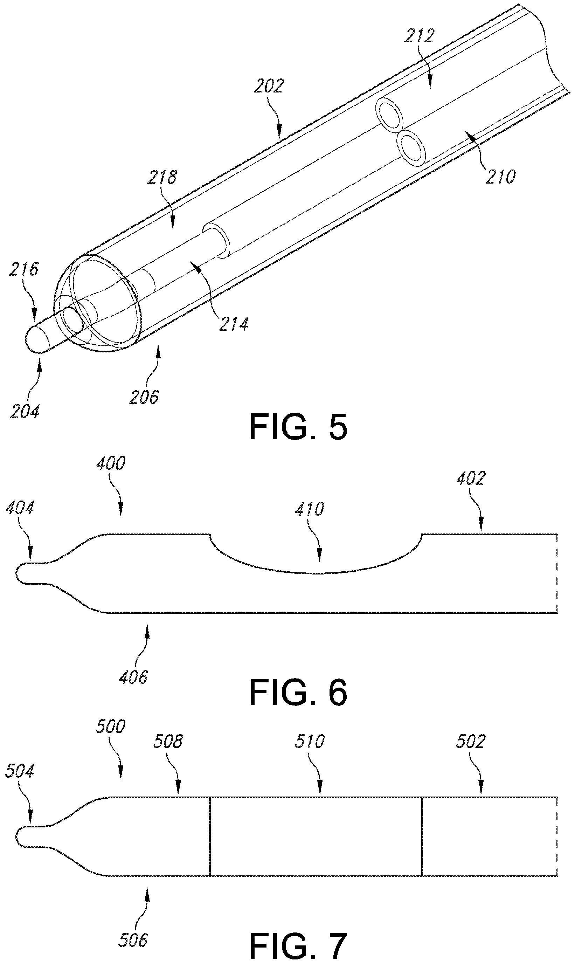

Turning now to FIG. 5, the internal components of the radiofrequency probe 200 are shown. Located within the elongated electrocap 202 at the distal end 206 of the probe 200 is an active tip 204 for providing the thermal and electrical or radiofrequency energy to the patient's target nerve location. The active tip 204 includes a thermocouple 216 extending from the end of a thermocouple hypotube 214 within the probe 200 at the active tip 204. Within the probe 200 are additionally a first fluid tubing 210 and a second fluid tubing 212 for carrying cooling fluid to and from the active tip 204 of the probe 200 and circulating within the fluid volume 218. The elongated electrocap 202 can extend continuously from the distal end 206 to the handle 220 of the probe 200, as shown in FIGs. 2A-C.

The curved probe 200 can have a diameter that is narrower than the prior art 17 gauge probe of FIG. 1B. For example, the curved probe 200 can have a diameter in a range from about 18 gauge to about 22 gauge, such as from about 19 gauge to about 21 gauge, e.g. 20 gauge. Importantly, the narrower probe diameter of the probe 200 as compared to the existing 17 gauge probe of FIG. 1B is much easier to achieve an angle of curvature, e.g. angle Θs as illustrated in FIG. 5, than curving a probe having a 17 gauge diameter. Within the curved probe 200, the first 210 and second 212 fluid tubing and the thermocouple hypotube 214 can be curved within the probe 200 at the same angle θ₃ after assembly of the fluid tubing 210, 212 and the hypotube 214 within the elongated electrocap 202.

The curved probe 200 is capable of creating lesions in patient tissue having comparable size to lesions created by a larger diameter, straight 17 gauge cooled radiofrequency probe when provided with the same power and settings from a radiofrequency generator. For example, Table 1 below shows the lesion height and lesion width of 11 sample lesions created by a 20 gauge cooled radiofrequency probe 200 at an average power of about 4.83 ± 0.79 watts. The sample lesions created were performed ex vivo in a raw chicken breast.

**Table 1**

| Sample | Lesion Height | Lesion Width |
|---|---|---|
| 1 | 12.3 | 11.31 |
| 2 | 9.94 | 9.14 |
| 3 | 12.04 | 12.59 |
| 4 | 9.21 | 9.99 |
| 5 | 10.12 | 9.18 |
| 6 | 11.57 | 12.09 |
| 7 | 9.84 | 10.04 |
| 8 | 11.84 | 10.9 |
| 9 | 10.56 | 10.28 |
| 10 | 10.42 | 10.7 |
| 11 | 10.42 | 10.03 |

The mean lesion height of the lesions described in Table 1 was 10.75091 mm with a standard deviation of 1.021454 mm, and the mean lesion width was 10.56818 mm with a standard deviation of 1.097003 mm. The lesions created using the 20 gauge curved cooled radiofrequency probe 200 are comparable in size to lesions created by a 17 gauge cooled radiofrequency probe. Thus, the 20 gauge curved probe significantly improves the steerability of the radiofrequency probe compared to the existing straight 17 gauge diameter probe while delivering the same lesion size for treatment of a patient's tissue.

FIG. 6 illustrates an embodiment of the present invention having a steerable probe end. Cooled radiofrequency probe 400 includes similar features as shown with respect to probe 200 in FIGs. 2C and 5, except the probe 400 does not have a curved distal end. For example, probe 400 includes an electrocap 402 having a distal end 406 including an active tip 404 for delivering cooled radiofrequency energy to patient tissue. The probe 400 additionally includes a cut-out section 410 of the electrocap 402 whereby the generally cylindrical-shaped electrocap 402 has an indentation, as illustrated in FIG. 6. The cut-out section 410 can enable the distal end 406 of the electrocap 402 to flex or bend in order to improve the steerability of the probe in tissue. The cut-out section 410 can have a shape as illustrated in FIG. 6 or any other cut-out shape that enables the probe 400 to flex or bend for improved steerability through the introducer 250, such as a V-shape, circle-shape, or other shape cut-out.

FIG. 7 illustrates yet another exemplary embodiment of the present invention having a steerable probe end. Cooled radiofrequency probe 500 includes similar features as shown with respect to probe 200 in FIGs. 2C and 5, except the probe 500 does not have a curved distal end. For example, probe 500 includes an electrocap 502 having a distal end 506 including an active tip 504 for delivering cooled radiofrequency energy to patient tissue. The electrocap 502 is separated into two sections, a proximal extended electrocap 502 that is configured to be connected to a probe handle (not shown) and a distal electrocap 508, where the distal end 506 and active tip 504 are part of the distal electrocap 508. A polymer section 510 is positioned between the proximal 502 and distal 508 sections of the electrocap 502. The polymer section 510 can enable the probe 500 to flex or bend in the polymer section 510 in order to improve steerability of the probe 500 in tissue. The polymer section 510 can be made from any number of polymer-based materials that possess a high dielectric and structural strength such as, but not limited to, fluorinated ethylene propylene (FEP), polytetrafluoroethylene (PTFE), high-density polyethylene (HDPE), and polyamide.

This written description uses examples to disclose the invention, including the best mode, and also to enable any person skilled in the art to practice the invention, including making and using any devices or systems and performing any incorporated methods. The patentable scope of the invention is defined by the claims.

## Claims

1. A cooled radiofrequency ablation probe (400, 500) comprising:
an electrocap assembly (402, 502) comprising an elongated member having a body, a proximal end (208) configured to interface with a probe handle (220), and a thermally and electrically conductive active tip (404, 504) at a distal end (406, 506) of the elongated member configured to deliver electrical or radiofrequency energy to a patient's tissue,
wherein the elongated member houses at least one cooling fluid tubing (210, 212) within the length of the body and a thermocouple hypotube (214) within the length of the elongated member,
wherein the body and the proximal end of the elongated member are straight,
further wherein the elongated member has a bendable portion (410, 510) between the distal end and the body such that the distal end of the elongated member is configured to flex or bend to improve steerability of the probe in the tissue.

2. The probe of claim 1, wherein the bendable portion comprises a cut-out section (410) such that the generally cylindrical-shaped electrocap assembly (402) has an indentation.

3. The probe of claim 2, wherein the cut-out section (410) has a shape that enables the distal end to flex or bend relative to the body of the elongated member.

4. The probe of claim 3, wherein the shape is a V-shape or circle-shape.

5. The probe of claim 1, wherein the bendable portion comprises a polymer section (510).

6. The probe of claim 1, wherein the body and the proximal end of the elongated member are electrically insulated.

7. The probe of claim 6, wherein a portion of the elongated member is not electrically insulated to expose the active tip (404, 504) for delivering electrical or radiofrequency energy.

8. The probe of claim 1, wherein the elongated member has a 20 gauge needle diameter.

9. A cooled radiofrequency ablation delivery kit (300), the kit comprising:
the cooled radiofrequency ablation probe (400, 500) according to any one of claims 1 to 8;
an introducer (250) having a hollow elongate member (256), the hollow elongate member having a distal end (252), a body, and a proximal end (254), wherein the hollow elongate member is straight from the distal end to the proximal end; and
a stylet (270) having a piercing surface on a tip of a distal end (272), wherein the stylet is configured to be inserted through the introducer to create a puncture wound in patient tissue,
wherein the probe (400, 500) is configured to be inserted through the introducer to deliver electrical or radiofrequency energy to the patient tissue via the active tip (404, 504) at the distal end (406, 506) of the probe.

10. The kit of claim 9, wherein the introducer (250) is configured to conform to the distal ends (272, 406, 506) of the stylet (270) and the probe (400, 500), respectively, when the stylet or the probe is inserted through the hollow elongate member (256).

11. The kit of claim 9, wherein the probe (400, 500) is longer than the stylet (270).

12. The kit of claim 9, wherein the stylet (270) and the probe (400, 500) are formed from a rigid material.

13. The kit of claim 9, wherein the introducer (250) comprises a male connector (260), further wherein each of the stylet (270) and the probe (400, 500) comprise female connectors (274, 222) for coupling to the male connector of the introducer.

## Patentansprüche

1. Gekühlte Funkfrequenz-Ablationssonde (400, 500), welche Folgendes umfasst:
eine Elektrokappenanordnung (402, 502), die ein längliches Element mit einem Körper, einem proximalen Ende (208), das so konfiguriert ist, dass es mit einem Sondengriff (220) zusammenwirkt, und einer thermisch und elektrisch leitenden aktiven Spitze (404, 504) an einem distalen Ende (406, 506) des länglichen Elements umfasst, das so konfiguriert ist, dass es elektrische oder Funkfrequenzenergie an das Gewebe eines Patienten abgibt, wobei das längliche Element mindestens einen Kühlflüssigkeitsschlauch (210, 212) innerhalb der Länge des Körpers und ein Thermoelement-Kanülenrohr (214) innerhalb der Länge des länglichen Elements beherbergt, wobei der Körper und das proximale Ende des länglichen Elements gerade sind, wobei ferner das längliche Element einen biegbaren Abschnitt (410, 510) zwischen dem distalen Ende und dem Körper aufweist, so dass das distale Ende des länglichen Elements so konfiguriert ist, dass es sich biegt oder krümmt, um die Steuerbarkeit der Sonde im Gewebe zu verbessern.

2. Die Sonde nach Anspruch 1, wobei der biegbare Teil einen ausgeschnittenen
Abschnitt (410) umfasst, so dass die allgemein zylindrisch geformte Elektrokappenanordnung (402) eine Einkerbung aufweist.

3. Die Sonde nach Anspruch 2, wobei der ausgeschnittene Abschnitt (410) eine Form hat, die es dem distalen Ende ermöglicht, sich relativ zum Körper des länglichen Elements zu biegen oder zu krümmen.

4. Die Sonde nach Anspruch 3, wobei die Form V-förmig oder kreisförmig ist.

5. Die Sonde nach Anspruch 1, wobei der biegsame Teil ein Polymer-Abschnitt (510) umfasst.

6. Die Sonde nach Anspruch 1, wobei der Körper und das proximale Ende des länglichen Elements elektrisch isoliert sind.

7. Die Sonde nach Anspruch 6, wobei ein Teil des länglichen Elements nicht elektrisch isoliert ist, um die aktive Spitze (404, 504) zur Abgabe von elektrischer oder Funkfrequenzenergie freizulegen.

8. Die Sonde nach Anspruch 1, wobei das längliche Element einen Nadeldurchmesser von 20 Gauge hat.

9. Ein gekühltes Funkfrequenz-Ablationsset (300), wobei das Set Folgendes umfasst:
die gekühlte Funkfrequenz-Ablationssonde (400, 500) nach einem der Ansprüche 1 bis 8;
eine Einführvorrichtung (250) mit einem hohlen länglichen Element (256), wobei das hohle längliche Element ein distales Ende (252), einen Körper und ein proximales Ende (254) aufweist, wobei das hohle längliche Element vom distalen Ende zum proximalen Ende gerade ist; und
ein Stilett (270) mit einer durchstechenden Oberfläche an einer Spitze eines distalen Endes (272), wobei das Stilett so konfiguriert ist, dass es durch die Einführvorrichtung eingeführt werden kann, um eine Punktionswunde im Patientengewebe zu erzeugen,
wobei die Sonde (400, 500) so konfiguriert ist, dass sie durch die Einführvorrichtung eingeführt werden kann, um über die aktive Spitze (404, 504) am distalen Ende (406, 506) der Sonde elektrische oder Funkfrequenzenergie an das Patientengewebe abzugeben.

10. Das Set nach Anspruch 9,
wobei die Einführvorrichtung (250) so konfiguriert ist, dass sie sich an die
distalen Enden (272, 406, 506) des Stiletts (270) bzw. der Sonde (400, 500) entsprechend anpasst, wenn das Stilett oder die Sonde durch das hohle, längliche Element (256) eingeführt werden.

11. Das Set nach Anspruch 9, wobei die Sonde (400, 500) länger ist als das Stilett (270).

12. Das Set nach Anspruch 9, wobei das Stilett (270) und die Sonde (400, 500) aus einem starren Material geformt sind.

13. Das Set nach Anspruch 9, wobei die Einführvorrichtung (250) einen männlichen Anschluss (260) umfasst, wobei ferner sowohl das Stilett (270) als auch die Sonde (400, 500) weibliche Anschlüsse (274, 222) zur Kopplung mit dem männlichen Anschluss der Einführvorrichtung umfassen.

## Revendications

1. Sonde d'ablation par radiofréquence refroidie (400, 500) comprenant :
un ensemble électrocap (402, 502) comprenant un élément allongé ayant un corps, une extrémité proximale (208) configurée pour faire interface avec une poignée de sonde (220), et une pointe active thermiquement et électriquement conductrice (404, 504) à une extrémité distale (406, 506) de l'élément allongé configurée pour administrer de l'énergie électrique ou radiofréquence à un tissu d'un patient,
dans laquelle l'élément allongé abrite au moins un tube de fluide de refroidissement (210, 212) sur la longueur du corps et un hypotube de thermocouple (214) sur la longueur de l'élément allongé,
dans laquelle le corps et l'extrémité proximale de l'élément allongé sont droits,
en outre, dans laquelle l'élément allongé comporte une partie pliable (410, 510) entre l'extrémité distale et le corps de telle sorte que l'extrémité distale de l'élément allongé soit configurée pour fléchir ou se plier afin d'améliorer la maniabilité de la sonde dans le tissu.

2. Sonde selon la revendication 1, dans laquelle la partie pliable comprend une section découpée (410) de telle sorte que l'ensemble électrocap de forme généralement cylindrique (402) présente une indentation.

3. Sonde selon la revendication 2, dans laquelle la section découpée (410) a une forme qui permet à l'extrémité distale de fléchir ou de se plier par rapport au corps de l'élément allongé.

4. Sonde selon la revendication 3, dans laquelle la forme est une forme de V ou une forme de cercle.

5. Sonde selon la revendication 1, dans laquelle la partie pliable comprend une section de polymère (510).

6. Sonde selon la revendication 1, dans laquelle le corps et l'extrémité proximale de l'élément allongé sont électriquement isolés.

7. Sonde selon la revendication 6, dans laquelle une partie de l'élément allongé n'est pas isolée électriquement pour exposer la pointe active (404, 504) pour administrer de l'énergie électrique ou radiofréquence.

8. Sonde selon la revendication 1, dans laquelle l'élément allongé a un diamètre d'aiguille de calibre 20.

9. Kit d'administration d'ablation par radiofréquence refroidi (300), le kit comprenant :
la sonde d'ablation par radiofréquence refroidie (400, 500) selon l'une quelconque des revendications 1 à 8 ;
un introducteur (250) ayant un élément allongé creux (256), l'élément allongé creux ayant une extrémité distale (252), un corps et une extrémité proximale (254), dans lequel l'élément allongé creux est droit depuis l'extrémité distale jusqu'à l'extrémité proximale ; et
un stylet (270) ayant une surface de perçage sur une pointe d'une extrémité distale (272), dans lequel le stylet est configuré pour être inséré à travers l'introducteur pour créer une plaie perforante dans un tissu du patient,
dans lequel la sonde (400, 500) est configurée pour être insérée à travers l'introducteur afin d'administrer une énergie électrique ou radiofréquence au tissu du patient via la pointe active (404, 504) à l'extrémité distale (406, 506) de la sonde.

10. Kit selon la revendication 9, dans lequel l'introducteur (250) est configuré pour se conformer aux extrémités distales (272, 406, 506) du stylet (270) et de la sonde (400, 500), respectivement, lorsque le stylet ou la sonde est inséré à travers l'élément allongé creux (256) .

11. Kit selon la revendication 9, dans lequel la sonde (400, 500) est plus longue que le stylet (270).

12. Kit selon la revendication 9, dans lequel le stylet (270) et la sonde (400, 500) sont formés à partir d'un matériau rigide.

13. Kit selon la revendication 9, dans lequel l'introducteur (250) comprend un connecteur mâle (260), dans lequel en outre chacun du stylet (270) et de la sonde (400, 500) comprend des connecteurs femelles (274, 222) pour le couplage au connecteur mâle de l'introducteur.
